# EUROPEAN PATENT APPLICATION

(11) **EP 0 764 722 A2**
(43) Date of publication of application: **26.03.1997**
(21) Application number: 96306721.0
(22) Date of filing: 16.09.1996
(51) Int. Cl.: C12N 15/16, C12N 15/70, C12N 1/21, C07K 14/575, A61K 38/22

(54) **DNA encoding primate leptins as medicinal proteins**

(30) Priority: 19.09.1995 US 3935; 19.09.1995 US 3936
(71) Applicant: ELI LILLY AND COMPANY, Indianapolis, Indiana 46285 (US)
(72) Inventor: Basinski, Margret Barbara, Indianapolis, Indiana 46219 (US); Hsiung, Hansen Maxwell, Carmel, Indiana 46032 (US); Schoner, Brigitte Elisabeth, Monrovia, Indiana 46157 (US); Zhang, Xing-Yue, Indianapolis, Indiana 46227 (US)
(74) Representative: Tapping, Kenneth George

(57) **Abstract**

The invention includes nucleic acid sequences encoding medically useful proteins, pharmaceutical formulations, and methods for expressing the proteins. In particular, higher primate leptin protein cDNA sequences are disclosed as are the expressed proteins.

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: DNA ENCODING MEDICINAL PROTEINS

(iii) NUMBER OF SEQUENCES: 8

(iv) CORRESPONDENCE ADDRESS:
(A) ADDRESSEE: K. G. Tapping
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1

(2) INFORMATION FOR SEQ ID NO:1:

(i) SEQUENCE CHARACTERISTICS:
(A) LENGTH: 438 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..438

(xi) SEQUENCE DESCRIPTION: SEQ ID NO:1:

GTG CCC ATC CAA AAA GTC CAG GAT GAC ACC AAA ACC CTC ATC AAG ACA 48
Val Pro Ile Gln Lys Val Gln Asp Asp Thr Lys Thr Leu Ile Lys Thr
1               5                   10                  15

ATT GTC ACC AGG ATC AAT GAC ATT TCA CAC ACG CAG TCA GTC TCC TCC 96
Ile Val Thr Arg Ile Asn Asp Ile Ser His Thr Gln Ser Val Ser Ser
            20                  25                  30

AAA CAG AAG GTC ACC GGT TTG GAC TTC ATT CCT GGG CTC CAC CCT ATC 144
Lys Gln Lys Val Thr Gly Leu Asp Phe Ile Pro Gly Leu His Pro Ile
        35                  40                  45

CTG ACC TTA TCC AAG ATG GAC CAG ACA CTG GCA GTC TAC CAA CAG ATC 192
Leu Thr Leu Ser Lys Met Asp Gln Thr Leu Ala Val Tyr Gln Gln Ile
    50                  55                  60

CTC ACC AGT ATG CCT TCC AGA AAC ATG ATC CAA ATA TCC AAC GAC CTG 240
Leu Thr Ser Met Pro Ser Arg Asn Met Ile Gln Ile Ser Asn Asp Leu
65                  70                  75                  80

GAG AAC CTC CGG GAC CTT CTT CAC GTG CTG GCC TTC TCT AAG AGC TGC 288
Glu Asn Leu Arg Asp Leu Leu His Val Leu Ala Phe Ser Lys Ser Cys
                85                  90                  95

CAC TTG CCC TGG GCC AGT GGC CTG GAG ACC TTG GAC AGC CTG GGG GGT 336
His Leu Pro Trp Ala Ser Gly Leu Glu Thr Leu Asp Ser Leu Gly Gly
            100                 105                 110

GTC CTG GAA GCT TCA GGC TAC TCC ACA GAG GTG GTG GCC CTG AGC AGG 384
Val Leu Glu Ala Ser Gly Tyr Ser Thr Glu Val Val Ala Leu Ser Arg
        115                 120                 125

CTG CAG GGG TCT CTG CAG GAC ATG CTG TGG CAG CTG GAC CTC AGC CCT 432
Leu Gln Gly Ser Leu Gln Asp Met Leu Trp Gln Leu Asp Leu Ser Pro
    130                 135                 140

GGG TGC 438
Gly Cys
145

(2) INFORMATION FOR SEQ ID NO:2:

(i) SEQUENCE CHARACTERISTICS:
(A) LENGTH: 146 amino acids
(B) TYPE: amino acid
(D) TOPOLOGY: linear

(ii) MOLECULE TYPE: protein

(xi) SEQUENCE DESCRIPTION: SEQ ID NO:2:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:3:

     (i) SEQUENCE CHARACTERISTICS:
          (A) LENGTH: 438 base pairs
          (B) TYPE: nucleic acid
          (C) STRANDEDNESS: single
          (D) TOPOLOGY: linear

    (ii) MOLECULE TYPE: cDNA


    (ix) FEATURE:
          (A) NAME/KEY: CDS
          (B) LOCATION: 1..438


    (xi) SEQUENCE DESCRIPTION: SEQ ID NO:3:

GTG CCC ATC CCA AAA GTC CAA GAT GAC ACC AAA ACC CTC ATC AAG ACA        48
Val Pro Ile Pro Lys Val Gln Asp Asp Thr Lys Thr Leu Ile Lys Thr
            150                 155                 160

ATT GTC ACC AGG ATC AGT GAC ATT TCA CAC ACG CAG TCA GTC TCC TCC        96
Ile Val Thr Arg Ile Ser Asp Ile Ser His Thr Gln Ser Val Ser Ser
        165                 170                 175

AAA CAG AAG GTC ACC GGT TTG GAC TTC ATT CCT GGG CTC CAC CCC ATC       144
Lys Gln Lys Val Thr Gly Leu Asp Phe Ile Pro Gly Leu His Pro Ile
    180                 185                 190

CTG ACC TTA TCC AAG ATG GAC CAG ACA CTG GCA GTC TAC CAA CAG ATC       192
Leu Thr Leu Ser Lys Met Asp Gln Thr Leu Ala Val Tyr Gln Gln Ile
195                 200                 205                 210

CTC ACC AGT ATG CCT TCC AGA AAC ATG ATC CAA ATA TCC AAC GAC CTG       240
Leu Thr Ser Met Pro Ser Arg Asn Met Ile Gln Ile Ser Asn Asp Leu
                215                 220                 225

GAG AAC CTC CGG GAC CTT CTT CAC GTG CTG GCC TTC TCT AAG AGC TGC       288
Glu Asn Leu Arg Asp Leu Leu His Val Leu Ala Phe Ser Lys Ser Cys
            230                 235                 240

CAC TTG CCC TGG GCC AGT GGC CTG GAG ACC TTG GAC AGC CTG GGG GGT       336
His Leu Pro Trp Ala Ser Gly Leu Glu Thr Leu Asp Ser Leu Gly Gly
        245                 250                 255

GTC CTG GAA GCT TCA GGC TAC TCC ACA GAG GTG GTG GCC CTG AGC AGG       384
Val Leu Glu Ala Ser Gly Tyr Ser Thr Glu Val Val Ala Leu Ser Arg
    260                 265                 270

CTG CAG GGG TCT CTG CAG GAC ATG CTG TGG CAG CTG GAC CTC AGC CCT       432
Leu Gln Gly Ser Leu Gln Asp Met Leu Trp Gln Leu Asp Leu Ser Pro
275                 280                 285                 290

GGG TGC                                                               438
Gly Cys

(2) INFORMATION FOR SEQ ID NO:4:

(i) SEQUENCE CHARACTERISTICS:
(A) LENGTH: 146 amino acids
(B) TYPE: amino acid
(D) TOPOLOGY: linear

(ii) MOLECULE TYPE: protein

(xi) SEQUENCE DESCRIPTION: SEQ ID NO:4:

Val Pro Ile Pro Lys Val Gln Asp Asp Thr Lys Thr Leu Ile Lys Thr
1               5                   10                  15

Ile Val Thr Arg Ile Ser Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:5:

(i) SEQUENCE CHARACTERISTICS:
(A) LENGTH: 438 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..438

(xi) SEQUENCE DESCRIPTION: SEQ ID NO:5:

GTG CCC ATC CAA AAA GTC CAA GAT GAC ACC AAA ACC CTC ATC AAG ACA 48
Val Pro Ile Gln Lys Val Gln Asp Asp Thr Lys Thr Leu Ile Lys Thr
150 155 160

GTT ATC ACC AGG ATC AAT GAC ATT TCA CAC ACG CAG TCA GTC TCC TCC 96
Val Ile Thr Arg Ile Asn Asp Ile Ser His Thr Gln Ser Val Ser Ser
165 170 175

AAA CAG AAG GTC ACC GGT TTG GAC TTC ATT CCT GGG CTC CAC CCC ATC 144
Lys Gln Lys Val Thr Gly Leu Asp Phe Ile Pro Gly Leu His Pro Ile
180 185 190

CTG ACC TTA TCC AAG ATG GAC CAG ACA CTG GCA GTC TAC CAA CAG ATC 192
Leu Thr Leu Ser Lys Met Asp Gln Thr Leu Ala Val Tyr Gln Gln Ile
195 200 205 210

CTC ACC AGT ATG CCT TCC AGA AAC GTG ATC CAA ATA TCC AAC GAC CTG 240
Leu Thr Ser Met Pro Ser Arg Asn Val Ile Gln Ile Ser Asn Asp Leu
215 220 225

GAG AAC CTC CGG GAC CTT CTT CAC GTG CTG GCC TTC TCT AAG AGC TGC 288
Glu Asn Leu Arg Asp Leu Leu His Val Leu Ala Phe Ser Lys Ser Cys
230 235 240

CAC TTG CCC TGG GCC AGT GGC CTG GAG ACC TTG GAC AGC CTG GGG GGT 336
His Leu Pro Trp Ala Ser Gly Leu Glu Thr Leu Asp Ser Leu Gly Gly
245 250 255

GTC CTG GAA GCT TCA GGC TAC TCC ACA GAG GTG GTG GCC CTT AGC AGG 384
Val Leu Glu Ala Ser Gly Tyr Ser Thr Glu Val Val Ala Leu Ser Arg
260 265 270

CTG CAG CGG TCT CTG CAG GAC ATG CTG TGG CAG CTG GAC CTC AGC CCT 432
Leu Gln Arg Ser Leu Gln Asp Met Leu Trp Gln Leu Asp Leu Ser Pro
275 280 285 290

GGG TGC 438
Gly Cys

(2) INFORMATION FOR SEQ ID NO:6:

(i) SEQUENCE CHARACTERISTICS:
(A) LENGTH: 146 amino acids
(B) TYPE: amino acid
(D) TOPOLOGY: linear

(ii) MOLECULE TYPE: protein

(xi) SEQUENCE DESCRIPTION: SEQ ID NO:6:

Val Pro Ile Gln Lys Val Gln Asp Asp Thr Lys Thr Leu Ile Lys Thr
1               5                   10                  15

Val Ile Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Arg Ser Leu Gln Asp Met Leu Trp Gln Leu Asp Leu Ser Pro
    130                 135                 140

Gly Cys
145

2) INFORMATION FOR SEQ ID NO:7:

     (i) SEQUENCE CHARACTERISTICS:
          (A) LENGTH: 30 base pairs
          (B) TYPE: nucleic acid
          (C) STRANDEDNESS: single
          (D) TOPOLOGY: linear

    (ii) MOLECULE TYPE: protein

    (xi) SEQUENCE DESCRIPTION: SEQ ID NO:7:

CCCAAGAAGC CCATCCTGGG AAGGAAAATG                                    30

(2) INFORMATION FOR SEQ ID NO:8:

     (i) SEQUENCE CHARACTERISTICS:
          (A) LENGTH: 30 base pairs
          (B) TYPE: nucleic acid
          (C) STRANDEDNESS: single
          (D) TOPOLOGY: linear

    (ii) MOLECULE TYPE: protein

    (xi) SEQUENCE DESCRIPTION: SEQ ID NO:8:

CTTGCAGGAA GAGTGACCTT CAAGGCCTCA                                    30

## Description

The invention belongs to the general field of molecular biology as applied to biopharmaceutical research and development. The invention includes isolated, naturally-occurring proteins that help regulate the body's volume of adipose tissue, as well as DNA encoding the proteins.

Obesity, especially upper body obesity, is a common and very serious public health problem in the United States and throughout the world. According to recent statistics, more than 25% of the U.S. population and 27% of the Canadian population are over weight. Kuczmarski, Amer. J. of Clin. Nut. 55: 495S - 502S (1992); Reeder *et al.,* Can. Med. Ass. J., 23: 226-233 (1992). Upper body obesity carries the highest risk factor known for Type II Diabetes and is a significant risk factor for cardiovascular disease and cancer as well. Recent cost estimates for medical complications associated with obesity are $150 billion world wide. The problem has now become so serious that the Surgeon General has begun a national initiative to combat obesity in America.

Hypertension, dyslipidemia, and insulin resistance are the primary pathologies associated with obesity. Many studies have demonstrated that weight reduction through diet and exercise dramatically improves these serious medical conditions. Unfortunately, obese individuals generally fail to significantly reduce their body mass through diet and exercise and have a near 95% failure rate. This failure may be due to genetically inherited factors that contribute to increased appetite, preference for high calorie foods, reduced physical activity, reduced lipolytic metabolism, and increased lipogenic metabolism. This indicates that people inheriting these genetic traits are prone to becoming obese regardless of their efforts to combat the condition. Therefore, new pharmacological agents that can reverse obesity in spite of genetic predisposition are needed.

The *ob /ob* mouse model of obesity and diabetes is known to carry an autosomal recessive trait linked to a mutation in the sixth chromosome. Recently, Zhang and coworkers published the positional cloning of a mouse gene linked to this condition. Zhang *et al.* Nature 372: 425-32 (1994). This report discloses a mouse cDNA sequence encoding a 167 amino acid protein that is expressed exclusively in adipose tissue and compares this mouse *ob* gene product (leptin) to a human homolog. The report also discloses a point mutation resulting in the conversion of an Arg codon to a stop codon at position 105. This mutant gene is postulated to expresses a truncated protein that lacks the biological function of the complete intact protein.

Physiologist have long postulated that excess fat cells laid down through overeating signals the brain that the body is obese which, in turn, causes the body to eat less and burn more fuel. G. R. Hervey, Nature 227: 629-631 (1969). Parabiotic experiments support a "feedback" model and suggest that a circulating peptide hormone may regulate the size of the body's fat depot. The newly disclosed *ob* gene product mentioned above is now believed to be such a hormone.

Recent reports have confirmed this hypothesis by demonstrating that treatment of obese mice that were either homozygous (*ob/ob*) or heterozygous (*+/?*) for the obese gene mutation with recombinant *ob* gene product lowered body weight, percent body fat, food intake, and serum concentrations of glucose and insulin. Pelleymounter *et al.*, Science 269: 540-543 (1995). Similar results were also reported by Halaas *et al.* Science 269: 543-546 (1995) and Campfield *et al.,* Science 269: 545-549 (1995).

The present invention is based on the discovery of obesity genes cloned from chimpanzee, gorilla, and orangutan genomic DNA. Therefore, this invention is useful for producing what is currently believed to be a biologically active proteins useful for treating obesity and reducing the risk for Type II diabetes, cardiovascular diseases, and cancer in mammals. It is also contemplated that conservative amino acid substitutions may be made to the protein in quiet regions without significantly altering biological activity. Consequently, analogous alterations at the DNA level to effect such substitutions are also contemplated.

The invention is also useful in immunogenicity animal models. In this mode, the invention can be used to predict the immunogenic capacity of analogs of human leptin. For example, if one wished to determine whether a specific substitution in human leptin would produce an antibody response in humans, the corresponding substitution is then made in one of the great ape ob proteins. This great ape analog is then injected into the corresponding ape species and its immunological response monitored. If the substitution failed to produce an immunological response, one would then accurately predict that a similar substitution in the human ob protein also would not raise a significant immunological response in humans.

The present invention is directed to isolated naturally occurring primate proteins that are biologically active, and DNA sequences that encode the proteins. The invention is also drawn to methods of treating obesity and diabetes, which comprises administering to a mammal in need thereof a protein of the invention. The invention further provides formulations, which comprise a protein of the invention, or a pharmaceutically acceptable thereof, together with one or more biologically acceptable diluents, carriers or excipients therefor.

For purposes of the present invention, as disclosed and claimed herein, the following terms and abbreviations are defined as follows:

Recombinant DNA Expression Vector -- any recombinant DNA cloning vector in which a promoter has been incorporated.

Nucleic acid molecule -- a DNA or RNA polymer.

Host Cell -- Any cell transformed using a recombinant DNA Vector and which is capable of either replicating or transcribing and translating DNA used to construct the recombinant DNA Vector.

Replicon -- A DNA sequence that controls and allows for autonomous replication of a plasmid or other vector.

Transcription -- the process whereby information contained in a nucleotide sequence of DNA is transferred to a complementary RNA sequence.

Translation -- the process whereby the genetic information of messenger RNA is used to specify and direct the synthesis of a polypeptide chain.

Treating -- describes the management and care of a human or veterinary patient for the purpose of combating a disease, condition, or disorder, of the patient. Treating includes the administration of a compound of present invention to prevent the onset of the symptoms or complications, alleviating the symptoms or complications, eliminating the disease, condition, or disorder. Treating therefor includes the inhibition of food intake, the inhibition of weight gain, and inducing weight loss in patients in need thereof.

Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

Amino acid and nucleotide abbreviations used herein are those accepted by the United States Patent and Trademark Office as set forth in 37 C.F.R. § 1.822 (b) (2) (1993). Unless otherwise indicated all amino acids are in the L configuration and protein sequences are listed beginning with the amino terminus. Nucleotide sequences are listed from 5' to 3'.

In one embodiment, the invention provides nucleic acid molecules that encode a novel chimpanzee *ob* gene product (chimp leptin) which is defined by the following amino acid sequence.

1                   5                   10                  15
Val Pro Ile Gln Lys Val Gln Asp Asp Thr Lys Thr Leu Ile Lys

                    20                  25                  30
Thr Ile Val Thr Arg Ile Asn Asp Ile Ser His Thr Gln Ser Val

                    35                  40                  45
Ser Ser Lys Gln Lys Val Thr Gly Leu Asp Phe Ile Pro Gly Leu

                    50                  55                  60
His Pro Ile Leu Thr Leu Ser Lys Met Asp Gln Thr Leu Ala Val

                    65                  70                  75
Tyr Gln Gln Ile Leu Thr Ser Met Pro Ser Arg Asn Met Ile Gln

                    80                  85                  90
Ile Ser Asn Asp Leu Glu Asn Leu Arg Asp Leu Leu His Val Leu

                    95                  100                 105
Ala Phe Ser Lys Ser Cys His Leu Pro Trp Ala Ser Gly Leu Glu

                    110                 115                 120
Thr Leu Asp Ser Leu Gly Gly Val Leu Glu Ala Ser Gly Tyr Ser

                    125                 130                 135
Thr Glu Val Val Ala Leu Ser Arg Leu Gln Gly Ser Leu Gln Asp

                    140                 145
Met Leu Trp Gln Leu Asp Leu Ser Pro Gly Cys-COOH

(SEQ ID NO: 2)

A preferred coding region for the above embodiment, chimp leptin, is defined by the following DNA sequence.

5'- GTG CCC ATC CAA AAA GTC CAG GAT GAC ACC AAA ACC CTC ATC
AAG ACA ATT GTC ACC AGG ATC AAT GAC ATT TCA CAC ACG CAG TCA
GTC TCC TCC AAA CAG AAG GTC ACC GGT TTG GAC TTC ATT CCT GGG
CTC CAC CCT ATC CTG ACC TTA TCC AAG ATG GAC CAG ACA CTG GCA
GTC TAC CAA CAG ATC CTC ACC AGT ATG CCT TCC AGA AAC ATG ATC
CAA ATA TCC AAC GAC CTG GAG AAC CTC CGG GAC CTT CTT CAC GTG
CTG GCC TTC TCT AAG AGC TGC CAC TTG CCC TGG GCC AGT GGC CTG
GAG ACC TTG GAC AGC CTG GGG GGT GTC CTG GAA GCT TCA GGC TAC
TCC ACA GAG GTG GTG GCC CTG AGC AGG CTG CAG GGG TCT CTG CAG
GAC ATG CTG TGG CAG CTG GAC CTC AGC CCT GGG TGC - 3'
(SEQ ID NO: 1)

In another embodiment, the invention provides nucleic acid molecules that encode a novel gorilla *ob* gene product (gorilla leptin) which is defined by the following amino acid sequence.

1                   5                   10                  15
Val Pro Ile Gln Lys Val Gln Asp Asp Thr Lys Thr Leu Ile Lys

                    20                  25                  30
Thr Ile Val Thr Arg Ile Ser Asp Ile Ser His Thr Gln Ser Val

                    35                  40                  45
Ser Ser Lys Gln Lys Val Thr Gly Leu Asp Phe Ile Pro Gly Leu

                    50                  55                  60
His Pro Ile Leu Thr Leu Ser Lys Met Asp Gln Thr Leu Ala Val

                    65                  70                  75
Tyr Gln Gln Ile Leu Thr Ser Met Pro Ser Arg Asn Met Ile Gln

                    80                  85                  90
Ile Ser Asn Asp Leu Glu Asn Leu Arg Asp Leu Leu His Val Leu

95                  100                 105
Ala Phe Ser Lys Ser Cys His Leu Pro Trp Ala Ser Gly Leu Glu

110                 115                 120
Thr Leu Asp Ser Leu Gly Gly Val Leu Glu Ala Ser Gly Tyr Ser

125                 130                 135
Thr Glu Val Val Ala Leu Ser Arg Leu Gln Gly Ser Leu Gln Asp

140                 145
Met Leu Trp Gln Leu Asp Leu Ser Pro Gly Cys-COOH

(SEQ ID NO: 4)

A preferred coding region for the above embodiment, gorilla leptin, is defined by the following DNA sequence.

5'- GTG CCC ATC CCA AAA GTC CAA GAT GAC ACC AAA ACC CTC ATC
AAG ACA ATT GTC ACC AGG ATC AGT GAC ATT TCA CAC ACG CAG TCA
GTC TCC TCC AAA CAG AAG GTC ACC GGT TTG GAC TTC ATT CCT GGG
CTC CAC CCC ATC CTG ACC TTA TCC AAG ATG GAC CAG ACA CTG GCA
GTC TAC CAA CAG ATC CTC ACC AGT ATG CCT TCC AGA AAC ATG ATC
CAA ATA TCC AAC GAC CTG GAG AAC CTC CGG GAC CTT CTT CAC GTG
CTG GCC TTC TCT AAG AGC TGC CAC TTG CCC TGG GCC AGT GGC CTG
GAG ACC TTG GAC AGC CTG GGG GGT GTC CTG GAA GCT TCA GGC TAC
TCC ACA GAG GTG GTG GCC CTG AGC AGG CTG CAG GGG TCT CTG CAG
GAC ATG CTG TGG CAG CTG GAC CTC AGC CCT GGG TGC - 3'
(SEQ ID NO: 3)

In yet another embodiment, the invention provides nucleic acid molecules that encode a novel orangutan *ob* gene product (orangutan leptin) which is defined by the following amino acid sequence.

1                   5                        10                       15
Val Pro Ile Gln Lys Val Gln Asp Asp Thr Lys Thr Leu Ile Lys

                    20                       25                       30
Thr Val Ile Thr Arg Ile Asn Asp Ile Ser His Thr Gln Ser Val

                    35                       40                       45
Ser Ser Lys Gln Lys Val Thr Gly Leu Asp Phe Ile Pro Gly Leu

50                  55                  60
His Pro Ile Leu Thr Leu Ser Lys Met Asp Gln Thr Leu Ala Val

                65                  70                  75
Tyr Gln Gln Ile Leu Thr Ser Met Pro Ser Arg Asn Val Ile Gln

                80                  85                  90
Ile Ser Asn Asp Leu Glu Asn Leu Arg Asp Leu Leu His Val Leu

                95                  100                 105
Ala Phe Ser Lys Ser Cys His Leu Pro Trp Ala Ser Gly Leu Glu

                110                 115                 120
Thr Leu Asp Arg Leu Gly Gly Val Leu Glu Ala Ser Gly Tyr Ser

                125                 130                 135
Thr Glu Val Val Ala Leu Ser Arg Leu Gln Arg Ser Leu Gln Asp

                140                 145
Met Leu Trp Gln Leu Asp Leu Ser Pro Gly Cys-COOH
(SEQ ID NO: 6)

A preferred coding region for the above embodiment, orangutan leptin, is defined by the following DNA sequence.

5'- GTG CCC ATC CAA AAA GTC CAA GAT GAC ACC AAA ACC CTC ATC
AAG ACA GTT ATC ACC AGG ATC AAT GAC ATT TCA CAC ACG CAG TCA
GTC TCC TCC AAA CAG AAG GTC ACC GGT TTG GAC TTC ATT CCT GGG
CTC CAC CCC ATC CTG ACC TTA TCC AAG ATG GAC CAG ACA CTG GCA
GTC TAC CAA CAG ATC CTC ACC AGT ATG CCT TCC AGA AAC GTG ATC
CAA ATA TCC AAC GAC CTG GAG AAC CTC CGG GAC CTT CTT CAC GTG
CTG GCC TTC TCT AAG AGC TGC CAC TTG CCC TGG GCC AGT GGC CTG
GAG ACC TTG GAC AGC CTG GGG GGT GTC CTG GAA GCT TCA GGC TAC
TCC ACA GAG GTG GTG GCC CTT AGC AGG CTG CAG CGG TCT CTG CAG
GAC ATG CTG TGG CAG CTG GAC CTC AGC CCT GGG TGC - 3'
(SEQ ID NO: 5)

The proteins of the present invention may be produced either by recombinant DNA technology or well known chemical procedures, such as solution or solid-phase peptide synthesis, or semi-synthesis in solution beginning with protein fragments coupled through conventional solution methods.

The principles of solid phase chemical synthesis of polypeptides are well known in the art and may be found in general texts in the area such as Dugas, H. and Penney, C., Bioorganic Chemistry Springer-Verlag, New York, pgs. 54-92 (1981). For example, peptides may be synthesized by solid-phase methodology utilizing an PE-Applied Biosystems 430A peptide synthesizer (commercially available from Applied Biosystems, Foster City California) and synthesis cycles supplied by Applied Biosystems. Boc amino acids and other reagents are commercially available from PE-Applied Biosystems and other chemical supply houses. Sequential Boc chemistry using double couple protocols are applied to the starting p-methyl benzhydryl amine resins for the production of C-terminal carboxamides. For the production of C-terminal acids, the corresponding PAM resin is used. Arginine, Asparagine, Glutamine, Histidine and Methionine are coupled using performed hydroxy benzotriazole esters. The following side chain protection may be used:
Arg, Tosyl
Asp, cyclohexyl or benzyl
Cys, 4-methylbenzyl
Glu, cyclohexyl
His, benzyloxymethyl
Lys, 2-chlorobenzyloxycarbonyl
Met, sulfoxide
Ser, Benzyl
Thr, Benzyl
Trp, formyl
Tyr, 4-bromo carbobenzoxy
Boc deprotection may be accomplished with trifluoroacetic acid (TFA) in methylene chloride. Formyl removal from Trp is accomplished by treatment of the peptidyl resin with 20% piperidine in dimethylformamide for 60 minutes at 4°C. Met(O) can be reduced by treatment of the peptidyl resin with TFA/dimethylsulfide/conHCl (95/5/1) at 25°C for 60 minutes. Following the above pre-treatments, the peptides may be further deprotected and cleaved from the resin with anhydrous hydrogen fluoride containing a mixture of 10% m-cresol or m-cresol/10% p-thiocresol or m-cresol/p-thiocresol/ dimethylsulfide. Cleavage of the side chain protecting group(s) and of the peptide from the resin is carried out at zero degrees Centigrade or below, preferably -20°C for thirty minutes followed by thirty minutes at 0°C. After removal of the HF, the peptide/resin is washed with ether. The peptide is extracted with glacial acetic acid and lyophilized. Purification is accomplished by reverse-phase C18 chromatography (Vydac) column in .1% TFA with a gradient of increasing acetonitrile concentration. One skilled in the art recognizes that the solid phase synthesis could also be accomplished using the FMOC strategy and a TFA/scavenger cleavage mixture.

The claimed DNA sequences are useful for expressing these primate *ob* gene products either by direct expression or as fusion proteins. When the claimed sequences are used in a fusion gene, the resulting product will require enzymatic or chemical cleavage. A variety of peptidases which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See U.S. Patent No. 5,126,249; Carter P., Site Specific Proteolysis of Fusion Proteins, Ch. 13 in Protein Purification: From Molecular Mechanisms to Large Scale Processes, American Chemical Soc., Washington, D.C. (1990).

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. The claimed protein is a relatively large protein. A synthetic coding sequence is designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The restriction sites are chosen so as to properly orient the coding sequence with control sequences to achieve proper in-frame reading and expression of the claimed protein.

In general, plasmid vectors containing promoters and control sequences which are derived from species compatible with the host cell are used with these hosts. The vector ordinarily carries a replication origin as well as marker sequences which are capable of providing phenotypic selection in transformed cells. For example, E. coli is typically transformed using pBR322, a plasmid derived from an E. coli species (Bolivar, et al., Gene 2: 95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. Patent No. 5,304,493, the teachings of which are herein incorporated by reference. The gene encoding A-C-B proinsulin described in U.S. Patent No. 5,304,493 can be removed from the plasmid pRB182 with restriction enzymes NdeI and BamHI. The claimed DNA sequences of the present invention can be inserted into the plasmid backbone on a NdeI/BamHI restriction fragment cassette.

In general, prokaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537). These examples are illustrative rather than limiting.

Prokaryotes also are used for expression. The aforementioned strains, as well as E. coli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the b-lactamase (vector pGX2907 [ATCC 39344] contains the replicon and b-lactamase gene) and lactose promoter systems (Chang et al., Nature, 275:615 (1978); and Goeddel et al., Nature 281:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

The claimed nucleic acid molecules may also be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. b-actin promoter. The early and late promoters of the SV40 virus are conveniently obtained as an SV40 restriction fragment which also contains the SV40 viral origin of replication. Fiers, et al., Nature, 273:113 (1978). The entire SV40 genome may be obtained from plasmid pBRSV, ATCC 45019. The immediate early promoter of the human cytomegalovirus may be obtained from plasmid pCMBb (ATCC 77177). Of course, promoters from the host cell or related species also are useful herein.

Transcription of the claimed DNA by higher eukaryotes is increased by inserting an enhancer sequence into the vector. Enhancers are cis-acting elements of DNA, usually about 10-300 bp, that act on a promoter to increase its transcription. Enhancers are relatively oriented and positioned independently and have been found 5' (Laimins, L. et al., PNAS 78:993 (1981)) and 3' (Lusky, M. L., et al., Mol. Cell Bio. 3:l108 (1983)) to the transcription unit, within an intron (Banerji, J. L. et al., Cell 33:729 (1983)) as well as within the coding sequence itself (Osborne, T. F., et al., Mol. Cell Bio. 4:1293 (1984)). Many enhancer sequences are now known from mammalian genes (globin, RSV, SV40, EMC, elastase, albumin, a-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BglII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R. C. and Berg, P. Science 209:1422 (1980), or hygromycin, Sugden, B. et al., Mol Cell. Biol. 5:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eukaryotic expression is pRc/CMV. pRc/CMV is commercially available from Invitrogen Corporation, 3985 Sorrento Valley Blvd., San Diego, CA 92121. To confirm correct sequences in plasmids constructed, the ligation mixtures are used to transform E. coli K12 strain DH10B (ATCC 31446) and successful transformants selected by antibiotic resistance where appropriate. Plasmids from the transformants are prepared, analyzed by restriction and/or sequence by the method of Messing, et al., Nucleic Acids Res. 9:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al., Molecular Cloning: A Laboratory Manual, Cold Spring Harbor Press, Cold Spring Harbor Laboratory, Cold Spring Harbor, New York (1989), or Current Protocols in Molecular Biology (1989) and supplements.

Preferred suitable host cells for expressing the vectors encoding the claimed proteins in higher eukaryotes include: African green monkey kidney line cell line transformed by SV40 (COS-7, ATCC CRL-1651); transformed human primary embryonal kidney cell line 293, (Graham, F. L. et al., J. Gen Virol. 36:59-72 (1977), Virology 77:319-329, Virology 86:10-21); baby hamster kidney cells (BHK-21(C-13), ATCC CCL-10, Virology 16:147 (1962)); chinese hamster ovary cells CHO-DHFR⁻ (ATCC CRL-9096), mouse Sertoli cells (TM4, ATCC CRL-1715, Biol. Reprod. 23:243-250 (1980)); african green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

In addition to prokaryotes, unicellular eukaryotes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., Nature 282:39 (1979); Kingsman et al., Gene 7:141 (1979); Tschemper et al., Gene 10:157 (1980)) is commonly used. This plasmid already contains the trp gene which provides a selection marker for a mutant strain of yeast lacking the ability to grow in tryptophan, for example ATCC no. 44076 or PEP4-1 (Jones, Genetics 85:12 (1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pACl ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which contain inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRyl21 ATCC 37658) utilization. Suitable vectors and promoters for use in yeast expression are further described in R. Hitzeman et al., European Patent Publication No. 73,657A. Yeast enhancers such as the UAS Gal from Saccharomyces cerevisiae (found in conjunction with the CYC1 promoter on plasmid YEpsec--hIlbeta ATCC 67024), also are advantageously used with yeast promoters.

The following examples will help describe how the invention is practiced and will illustrate the characteristics of the claimed DNA molecules, vectors, host cells, and methods of the invention.

### Example 1

### Chimpanzee Leptin DNA

Chimpanzee genomic DNA was purchased from BIOS Laboratories, New Haven, CT (Catalog No: DEP-100, lot 561). DNA primers were prepared for use in polymerase chain reaction (PCR) amplification methods using a Model 380A DNA synthesizers (PE-Applied Biosystems, Inc., 850 Lincoln Center Drive, Foster City, CA 94404). Primers sequences were as follows:

Set2.F (12609) CCC AAG AAG CCC ATC CTG GGA AGG AAA ATG
(SEQ. ID. NO:7)

Set2.R (12610) CTT GCA GGA AGA GTG ACC TTC AAG GCC TCA
(SEQ. ID. NO:8)

For amplification of chimpanzee genomic DNA, the polymerase chain reaction was carried out by preparing a 100 µL mixture of the above chimpanzee genomic DNA (1 Iµ, 0.5 µg/µL), 10 µl of 10x PCR reaction buffer (Perkin-Elmer Corporation), 4 µL of 10 mM dNTPs and 50 pmol each of the sense (Set2.F) and antisense (Set2.R) primers for Chimpanzee leptin DNA amplification. The PCR conditions were 95°C for 1 minute, 80°C for 5 minutes. During the 5 minute time period, rTth DNA polymerase (2 units, 1 µ) (Perkin-Elmer Corporation) was added to the PCR reaction mixture. Using a PCR DNA Thermocycler® (Perkin Elmer Corporation Model 9600), 16 PCR cycles were run at 94°C for 30 seconds, 57°C for 10 seconds, and 72°C for 5 minutes. Immediately after, 18 more cycles were run at 94°C for 30 seconds and 68°C for 6 minutes.

After PCR amplification, 5 µL BRL T4 DNA polymerase (5 U/µL), 2 µL BRL T4 polynucleotide kinase (10 U/µ), and 5 µL ATP (10 mM) were added to the PCR reaction mixture (100 µl) directly and incubated for 30 minutes at 37°C. After the incubation the reaction mixture was heated at 95°C for 5 minutes and then was cooled on ice. The 2700 bp fragment (∾0.5 µg) was purified by agarose gel electrophoresis and isolated by the freeze-squeeze method. The 2700 bp fragment (∾0.2 µg) was then ligated into SmaI linearized pUC18 plasmid (∾1 µg) and the ligation mixture was used to transform DH5α (BRL) competent cells. The transformation mixture was plated on 0.02% X-Gal TY broth plates containing ampicillin (Amp) (100 µg/mL) and was then incubated overnight at 37°C. White clones were picked and grown at 37°C overnight in TY broth containing Amp (100 µg/mL). Plasmid DNA was isolated using a Wizard Miniprep DNA purification system (Promega) and sequenced using an Applied Biosystem 370 DNA sequencer. The chimpanzee DNA sequence obtained by PCR was compared to the homologous human leptin DNA sequence to determine the intron-exon junctions. The coding region for the mature chimpanzee leptin is defined herein as SEQ ID NO:1. The native chimpanzee leptin protein sequence was deduced from the cloned DNA and is set forth as SEQ. ID. NO:2.

### Example 2

### Gorilla Leptin DNA

Gorilla genomic DNA was purchased from BIOS Laboratories, New Haven, CT (Catalog No: DEP-100, lot 561). Genomic cloning was conducted in substantial accordance with Example 1 and the primer sequences used were identical to those used in Example 1.

The gorilla DNA sequence obtained by PCR was compared to the homologous human leptin DNA sequence to determine the intron-exon junctions. The coding region for the mature gorilla leptin is defined herein as SEQ ID NO:3. The native gorilla leptin protein sequence was deduced from the cloned DNA and is set forth as SEQ. ID. NO:4.

### Example 3

### Orangutan Leptin DNA

Orangutan genomic DNA was purchased from BIOS Laboratories, New Haven, CT (Catalog No: DEP-100, lot 561). Genomic cloning was conducted in substantial accordance with Examples 1 and 2 and the primer sequences used were identical to those used in Example 1.

The orangutan DNA sequence obtained by PCR was compared to the homologous human leptin DNA sequence to determine the intron-exon junctions. The coding region for the mature gorilla leptin is defined herein as SEQ ID NO:5. The native gorilla leptin protein sequence was deduced from the cloned DNA and is set forth as SEQ. ID. NO:6.

### Example 4

### Vector Construction

A plasmid containing a cDNA, synthetic, or semisynthetic DNA sequence encoding the desired claimed protein is constructed to include NdeI and BamHI restriction sites. The plasmid carrying the DNA sequence of interest is digested with MunI and BamHI restriction enzymes. The small ∼ 400 bp fragment is gel-purified. The synthetic oligonucleotide linker is used to reconstruct the front part of the DNA up to MunI site and to introduce NdeI restriction sight and codons for Met-Arg. The synthetic linker, the 400 bp fragment, and pBR182 vector whose A-C-B proinsulin coding region is absent are ligated in a three piece ligation.

The ligation products are transformed into E. coli DH10B (commercially available from GIBCO-BRL) and colonies growing on tryptone-yeast (DIFCO) plates supplemented with 10 mg/mL of tetracycline are analyzed. Plasmid DNA is isolated, digested with NdeI and BamHI and the resulting fragments are separated by agarose gel electrophoresis. Plasmids containing the expected ∼ 400bp NdeI to BamHI fragment are kept. E. coli K12 RV308 (available from the NRRL under deposit number B-15624) are transformed with this second plasmid, resulting in a culture suitable for expressing the protein.

The techniques of transforming cells with the aforementioned vectors are well known in the art and may be found in such general references as Maniatis, et al. (1988) Molecular Cloning: A Laboratory Manual, Cold Spring Harbor Press, Cold Spring Harbor Laboratory, Cold Spring Harbor, New York or Current Protocols in Molecular Biology (1989) and supplements. The techniques involved in the transformation of E. coli cells used in the preferred practice of the invention as exemplified herein are well known in the art. The precise conditions under which the transformed E. coli cells are cultured is dependent on the nature of the E. coli host cell line and the expression or cloning vectors employed. For example, vectors which incorporate thermoinducible promoter-operator regions, such as the c1857 thermoinducible lambda-phage promoter-operator region, require a temperature shift from about 30 to about 40 degrees C. in the culture conditions so as to induce protein synthesis.

In the preferred embodiment of the invention, E. coli K12 RV308 cells are employed as host cells but numerous other cell lines are available such as, but not limited to, E. coli K12 L201, L687, L693, L507, L640, L641, L695, L814 (E. coli B). The transformed host cells are then plated on appropriate media under the selective pressure of the antibiotic corresponding to the resistance gene present on the expression plasmid. The cultures are then incubated for a time and temperature appropriate to the host cell line employed.

Proteins which are expressed in high-level bacterial expression systems characteristically aggregate in granules or inclusion bodies which contain high levels of the overexpressed protein. Kreuger et al., in Protein Folding, Gierasch and King, eds., pgs 136-142 (1990), American Association for the Advancement of Science Publication No. 89-18S, Washington, D.C. Such protein aggregates must be solubilized to provide further purification and isolation of the desired protein product. Id. A variety of techniques using strongly denaturing solutions such as guanidinium-HCl and/or weakly denaturing solutions such as dithiothreitol (DTT) are used to solubilize the proteins. Gradual removal of the denaturing agents (often by dialysis) in a solution allows the denatured protein to assume its native conformation. The particular conditions for denaturation and folding are determined by the particular protein expression system and/or the protein in question.

Preferably, the present DNA sequences are expressed with a dipeptide leader sequence encoding Met-Arg or Met-Tyr as described in U.S. Patent No. 5,126,249, herein incorporated by reference. This approach facilitates the efficient expression of proteins and enables rapid conversion to the active protein form with Cathepsin C or other dipeptidylaminopeptidases. The purification of proteins is by techniques known in the art and includes reverse phase chromatography, affinity chromatography, and size exclusion.

### EXAMPLE 3

### In Vivo Testing

Proteins of the present invention are tested for biological activity by injecting them (i.v., s.c., i.m., i.p., or by minipump or cannula) into various strains of mice and then monitoring food and water consumption, body weight gain, plasma chemistry or hormones (glucose, insulin, ACTH, corticosterone, GH, T4) over various time periods.

Suitable test animals include normal mice (ICR, etc.) and obese mice (*ob/ob,* Avy/a, KK-Ay, tubby, fat). The *ob/ob* mouse model of obesity and diabetes is generally accepted in the art as being indicative of the obesity condition. Controls for non-specific effects for these injections are done using vehicle with or without the active agent of similar composition in the same animal monitoring the same parameters or the active agent itself in animals that are thought to lack the receptor (db/db mice, fa/fa or cp/cp rats). Proteins demonstrating activity in these models will demonstrate similar activity in other mammals, particularly humans.

Since the target tissue is expected to be the hypothalamus where food intake and lipogenic state are regulated, a similar model is to inject the test article directly into the brain (e.g. i.c.v. injection via lateral or third ventricles, or directly into specific hypothalamic nuclei (e.g. arcuate, paraventricular, perifornical nuclei). The same parameters as above could be measured, or the release of neurotransmitters that are known to regulate feeding or metabolism could be monitored (e.g. NPY, galanin, norepinephrine, dopamine, b-endorphin release).

Similar studies are accomplished *in vitro* using isolated hypothalamic tissue in a perfusion or tissue bath system. In this situation, the release of neurotransmitters or electrophysiological changes is monitored.

Proteins of the present invention are believed to be active in at least one of the above biological tests. As such, the protein compositions are useful for treating obesity, diabetes, and disorders implicated by obesity. The claimed proteins sequences are also useful for preparing immunogens to raise antibodies for purification and diagnostic uses.

## Claims

1. An isolated nucleic acid molecule consisting of a nucleotide sequence that encodes a protein selected from the group consisting of SEQ. ID. NO:2, SEQ. ID. NO:4, and SEQ. ID. NO:6.

2. An isolated nucleic acid molecule of **Claim 1** consisting of a DNA sequence selected from the group consisting of SEQ. ID. NO:1, SEQ. ID. NO:3, and SEQ. ID. NO:5.

3. A vector comprising a nucleic acid molecule of **Claim 1**.

4. A vector comprising a nucleic acid molecule of **Claim 2**.

5. A recombinant host cell comprising a vector of **Claim 3**.

6. A recombinant host cell comprising a vector of **Claim 4**.

7. A process for producing an anti-obesity protein comprising;
a) culturing a host cell of **Claim 5** under conditions resulting in the expression of a protein selected from the group consisting of SEQ. ID. NO:2, SEQ. ID. NO:4, and SEQ. ID. NO:6; and,
b) isolating the protein expressed in step a.

8. A process for producing an anti-obesity protein comprising;
a) culturing a host cell of **Claim 6** under conditions resulting in the expression of a protein selected from the group consisting of SEQ. ID. NO:2, SEQ. ID. NO:4, and SEQ. ID. NO:6; and,
b) isolating the protein expressed in step a.

9. An isolated protein comprising an amino acid sequence selected from the group consisting of SEQ. ID. NO:2, SEQ. ID. NO:4, and SEQ. ID. NO:6 or a pharmaceutically acceptable salt thereof.

10. A protein of **Claim 9** which comprises SEQ. ID. NO:2.

11. A protein of **Claim 9** which comprises SEQ. ID. NO:4.

12. A protein of **Claim 9** which comprises SEQ. ID. NO:6.

13. A pharmaceutical formulation, which comprises a protein of **Claim 9**, or a pharmaceutically acceptable salt thereof, together with one or more pharmaceutically acceptable diluents, carriers or excipients therefor.

14. A pharmaceutical formulation, which comprises a protein of **Claim 10**, or a pharmaceutically acceptable salt thereof, together with one or more pharmaceutically acceptable diluents, carriers or excipients therefor.

15. A pharmaceutical formulation, which comprises a protein of **Claim 11**, or a pharmaceutically acceptable salt thereof, together with one or more pharmaceutically acceptable diluents, carriers or excipients therefor.

16. A pharmaceutical formulation, which comprises a protein of **Claim 12**, or a pharmaceutically acceptable salt thereof, together-with one or more pharmaceutically acceptable diluents, carriers or excipients therefor.

17. A protein, or a pharmaceutically acceptable salt thereof, as claimed in any one of **Claims 9 to 12**, for use in treating obesity.

18. A protein, or a pharmaceutically acceptable salt thereof, as claimed in any one of **Claims 9 to 12**, for use in treating diabetes.
